# EUROPEAN PATENT APPLICATION

(11) **EP 3 622 977 A1**
(43) Date of publication of application: **18.03.2020**
(21) Application number: 18194081.8
(22) Date of filing: 12.09.2018
(51) Int. Cl.: A61M 1/06

(54) **BREAST PUMP DEVICE**

(71) Applicant: Koninklijke Philips N.V., 5656 AG Eindhoven (NL)
(72) Inventor: The designation of the inventor has not yet been filed
(74) Representative: Roche, Denis

(57) **Abstract**

A breast pump device (100) is provided with a breast receiving portion (130), a pressure source (120) for generating an over- or under pressure, a controller (110) and a breast detector (140) for detecting a first state when no breast is in the breast receiving portion and a second state when a breast is in the breast receiving portion (130). The controller (110) changes a parameter of the breast pump device and/or outputs a notification if the breast detector (140) has detected the second state. Hence, the controller of the breast pump can take an action when the breast has been detected in the breast receiving portion.

## Description

### FIELD OF THE INVENTION

The invention relates to a breast pump device and a method of operating a breast pump device.

### BACKGROUND OF THE INVENTION

Breast pumps are used by breast feeding women to extract milk from their breast such that the extracted milk can be fed to their babies at a later time. Typically, the breast is placed into a funnel shaped cup and a vacuum is applied such that milk is extracted. The breast pump typically has two operating modes, namely a stimulation mode and an extraction mode. During the stimulation mode, the milk ejection reflex is stimulated. When the user is only placing one breast into the breast cup, the user can use the other hand to start or activate the breast pump. However, if the user is placing both their breasts into the breast cups, the user does not have any hand free for activating the breast pump. When the breast pump is activated, a pressure source like a vacuum pump inside the breast pump will generate a vacuum and the stimulation mode can be started.

It is, however, sometimes difficult for the user to correctly position the breast inside the breast receiving funnel of the breast pump before activating the breast pump. If two hands are required to hold the breast receiving funnel then it can be difficult to activate the pressure source. On the other hand, if the pressure source is activated at first than it may be difficult to position the breast receiving or funnel.

US 2011/004154 A1 describes a breast pump having a sensor for measuring a parameter of the pump during the use of the breast pump. The sensor can be a pressure sensor or a milk flow sensor. The pressure sensor is adapted to measure a negative pressure in a breast cup. The milk flow sensor is adapted to measure a milk flow. If the pressure measured by the pressure sensor differs from a predetermined pressure, a controller can adjust the settings of the breast pump.

### SUMMARY OF THE INVENTION

It is therefore an object of the invention to provide a breast pump device which improves the usability of the breast pump at the beginning of the operation of the breast pump.

This object is solved by a breast pump device which has at least one breast receiving portion for receiving a breast of a user. The breast pump device furthermore comprises a pressure source coupled to the at least one breast pump receiving portion. The pressure source is capable of creating an overpressure or an underpressure (vacuum) when the breast is placed into the at least one breast receiving portion. The pressure source can be operated in a variety of operating modes, for example a first operating mode (e.g. a detection mode or an off mode) and a second operating mode corresponding e.g. to a stimulation mode or an extraction mode. The breast pump device furthermore comprises a controller for controlling the operation of the pressure source. The breast pump device furthermore comprises a breast detector coupled to the controller. The breast detector detects a first state when no breast is placed in the at least one breast receiving portion or a second state when the breast is placed into the at least one breast receiving portion based on operating parameters of the pressure source like a vacuum source. The breast detector communicates the presence of the first or second state to the controller. The controller may initiate an action based on the detection of the first or second state by the breast detector. Hence, the controller of the breast pump can take an action when the breast has been detected in the breast receiving portion.

According to an embodiment of the invention the action taken by the controller can be a visual or audible indication of the state, a change in an operating parameter of the device or the storage of the information of the state-change. The controller can be configured to change a parameter of the breast pump device and/or to output a notification if the breast detector has detected the second state. Such a notification may also include a signal or command or an entry in a log or in a memory.

According to an embodiment of the invention, the controller operates the pressure source in the first operating mode or the second operating mode based on the detection of the first or second state by the breast detector.

According to an embodiment of the invention, the breast detector is capable of detecting the first or second state based on operating parameters of the pressure source.

According to an embodiment of the invention, the operating parameters of the pressure source can be the speed or acceleration of a motor of the pressure source, voltage or current measurements at the pressure source or the underpressure or overpressure created by the pressure source.

According to an embodiment of the invention, the operating parameters can be analyzed over time.

According to an embodiment of the invention, the operating parameters of the pressure source are chosen in such a way that the presence of a breast causes a detectable change in at least one parameter measurable by the breast detector.

According to an embodiment of the invention, the breast detector can also take into account leakage which occurs when the breast is placed into the breast receiving portion and a vacuum is applied.

According to an embodiment of the invention, when a breast pump is activated, reference measurements of the operating parameters of the vacuum source can be conducted.

According to an embodiment of the invention, the controller can control the vacuum source in an initial placement mode where the vacuum source applies a low vacuum such that the expression kit (the breast receiving portion) can be easily positioned over the breast thus enabling a detection mode for the detection of a breast in the breast receiving portion.

According to a further embodiment of the invention, if the breast detector does not detect the second state, the controller can initiate the second operating mode in order to start the stimulation or extraction mode. The stimulation mode and/or the extraction mode can also be a sequence of different operating modes in order to extract milk.

According to a further embodiment of the invention, the controller can operate a second pressure source based on the input from a detector for a first pressure source.

According to an embodiment of the invention, the controller operates the pressure source in a detection mode as the first mode, wherein the pressure source generates a low vacuum. The controller is configured to operate the pressure source in the second operating mode for milk expression

According to an embodiment of the invention the controller initiates the second operating mode of the pressure source based on an input from the user, based on an external signal or if the breast detector has not detected the second state after a predefined period of time.

According to an embodiment of the invention the breast pump comprises at least one breast receiving portion for receive a breast of a user, a pressure source coupled to the at least one breast receiving portion and being configured to generate an overpressure or an underpressure according to at least a first operating mode or a second operating mode, a controller configured to control an operation of the pressure source according to the first or second operating mode, a breast detector coupled to the controller and being configured to detect a first state when no breast is placed in the at least one breast receiving portion and to detect a second state when the breast is placed into the at least one breast receiving portion or to detect a third state when the breast is not placed correctly into the breast receiving portion such that leakage occurs based on operating parameters of the pressure source and to communicate the first, second or third state to the controller. The controller is configured to operate the pressure source in a first operating mode if the first state has been detected or a second operating mode if the second state has been detected. The controller is configured to operate the pressure source in a third operating mode if the third state has been detected.

According to an embodiment of the invention the controller changes a parameter of the breast pump device and/or outputs a notification, signal or command if the breast detector has detected the third state.

According to an embodiment of the invention the breast detector is configured to detect the third state based on operating parameters of the pressure source.

According to an embodiment of the invention the controller is configured to initiate a different sequence of modes or program than the original mode or sequence of modes after an interruption.

According to an embodiment of the invention the pressure source can be implemented as a hydraulic pump or as an electrical pump.

According to an embodiment of the invention the breast receiving portion is configured to be mechanically movable or flexible such that the breast inside the breast receiving portion can be massaged to improve the milk extraction.

Further aspects of the invention are defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 discloses a block diagram of a breast pump device according to an embodiment of the invention,
Fig. 2 shows a flow chart of the operation of the breast pump device according to an embodiment of the invention,
Fig. 3 shows a schematic representation of a breast pump device according to an embodiment of the invention, and
Fig. 4 shows a graph depicting a behavior of a vacuum pump according to an embodiment of the invention.

### DETAILED DESCRIPTION OF EMBODIMENTS

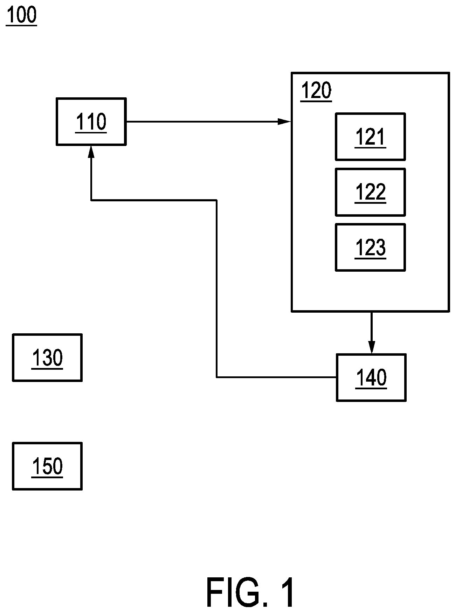

Fig. 1 discloses a block diagram of a breast pump device according to an embodiment of the invention. The breast pump device 100 comprises a controller 110, a pressure source 120 configured to generate an overpressure or an underpressure (vacuum source) 120, at least one breast receiving portion 130, a breast detector 140 and a milk extraction path 150. The pressure source 120 can comprise a motor 121 for creating an overpressure or an underpressure (e.g. a vacuum). The pressure source 120 may optionally comprise a sensor capable of measuring a characteristic of the pressure sources such as voltage or current or pressure and flow. Such a sensor can be a speed sensor 122 and a voltage or current sensor 123. The speed sensor 122 can be used to detect the speed of a motor 121 of the pressure source 120. The voltage or current sensor 122 can be used to detect the voltage or current of the motor 121.

According to an embodiment of the invention, the pressure source 120 is implemented as a vacuum source generating an underpressure.

The breast detector 140 is used to detect whether a breast is placed into one of the breast receiving portions. If the breast detector 140 does not detect a breast inside the breast receiving portion 130, the breast detector 140 will indicate a first state (no breast inside the breast detector to the controller 110. When the breast detector 140 detects the presence of a breast in one of the breast receiving portions 130, the breast detector 130 can communicate the second state to the controller 110.

According to an embodiment of the invention, the breast detector 140 uses operating parameters of the pressure source, like a vacuum source in order to detect the presence of a breast in the breast receiving portion 130. Hence, the breast detector 140 can differentiate between a first state (no breast) and a second state (breast in the breast receiving portion).

The operating parameters of the pressure source, like a vacuum source 120 can be the speed or acceleration of the motor 121 or the voltage or current of the motor 121.

The controller 110 will activate the second operating mode (stimulation mode) of the pressure source, like a vacuum source 120 when the breast detector 140 has indicated the second state. However, if the breast detector 140 does not indicate the second state but the first state, the controller 110 will not initiate the second operating mode of the pressure source, like a vacuum source 120.

Optionally, the pressure source, like a vacuum source 120 can have a detection mode as first operating mode. The detection mode may be activated by the controller 110 when the breast pump 100 is switched on. In the detection mode, the pressure source, like a vacuum source 120 can be optionally used to create a low vacuum such that the user can place the breast into the breast receiving portion without having the difficulties of coping with a high vacuum which can make the movement of the breast inside the breast receiving portion 130 difficult. In other words, during the detection mode, a low vacuum is applied by the pressure source, like a vacuum source to enable a comfortable placement of the breast inside the breast receiving portion. Alternatively, the change to a second operating mode as initiated when second state is detected can be delayed to allow additional time for a user to properly position the breast into the breast receiving portion.

The breast pump 100 can have a milk extraction path 150 which can include the breast receiving portion 140 (expression kit or breast cup). The milk extraction path 150 is the path the extracted milk follows until it flows into a bottle which can be attached to the breast pump device. The breast receiving portion 130 can be funnel shaped.

The controller 110 can activate the second operating mode (stimulation mode) when the breast detector 130 has detected the presence of a breast in the breast receiving portion 130. Thereafter, in the second operating mode, an extracting mode can be initiated by the controller 110 in order to extract milk from the breast of a user.

According to an embodiment of the invention, the breast detector 140 can be implemented as a dedicated unit. Alternatively, the controller 110 can perform at least some of the functions of the breast detector.

The pressure source can be implemented as an electrical pump or as a hydraulic pump. Optionally, the breast receiving portion is configured to be mechanically movable or flexible such that the breast inside the breast receiving portion can be massaged to improve the milk extraction.

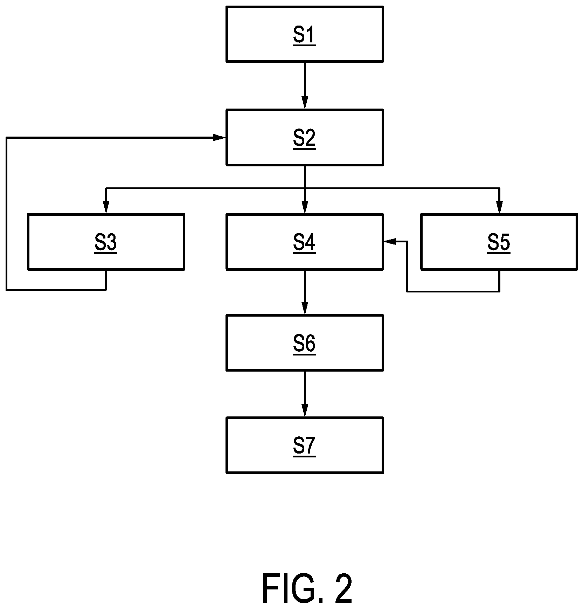

Fig. 2 shows a flow chart of the operation of the breast pump device according to an embodiment of the invention. In step S1, the breast pump is in the off-state. In step S2, the breast pump is switched on and a vacuum test (detection mode) is initiated. In other words, the breast detector 140 detects whether a breast is present in the breast receiving portion 130. Hence, the controller 110 may initiate the detection mode. If no breast has been detected in the breast receiving portion 130 by the breast detector 140, the flow continues to step S3, returning the flow to step S2 to perform another vacuum test (by initiating the detection mode). If, however, a breast is detected in the breast receiving portion 130 (second state, step S4), the expression sequence (second operating mode of the vacuum source) is initiated in step S6. If after a predetermined period of time the breast detector has not detected a breast in the breast receiving portion 130, then the controller can initiate the second operating mode. Alternatively, the controller may initiate the second operating mode based on an input of the user or based on an external signal. In step S7, the vacuum is reduced by the pressure source, like a vacuum source 120 and the breast pump 100 can be deactivated as the milk expression has ended.

If after a predefined time period the breast detector is still detecting the second state, the flow can continue to step S4 and the controller 110 can activate the second operating mode of the pressure source, like a vacuum source 120 to start the milk expression.

According to an embodiment of the invention, the breast detector 140 uses operating parameters of the pressure source, like a vacuum source to determine a first or second state, namely whether or not a breast is placed into the breast receiving portion 130. The operating parameter can be the voltage or current of the pressure source, like a vacuum source and in particular of a motor 121 of the pressure source like a vacuum source. The controller 110 will first of all only initiate the second operating mode (stimulating mode or the expression mode of the pressure source, like a vacuum source 120) if the breast detector 140 has detected a breast in one of the breast receiving portions 130. When after a certain time period no breast is detected in the other breast receiving portion, the controller 110 can switch from stimulation to expression mode or vice versa (both second operating mode). Alternatively, the controller 110 may switch from the second operating mode to the first operating mode.

The breast detector 140 can optionally take leakage of vacuum into account (for example as the breast may not be exactly positioned inside the breast receiving portion).

According to an embodiment of the invention, the detecting mode of the pressure source, like a vacuum source 120 can be initiated by the controller 110 when the breast pump is activated. The detection mode can be used until a breast is detected inside the breast receiving portion. In addition, the detection mode can also be used after a breast has been detected in the breast receiving portion (e.g. at intervals) as an in-between measurement during the second operating mode in order to determine whether the breast is still inside the breast receiving portion or to determine whether the breast is placed into the breast receiving portion and that there is no or little leakage.

According to an embodiment of the invention, the breast detector 140 may operate continuously or at intervals. The breast detector 140 can also determine a leakage as well as a pausing of the pressure source, like a vacuum source. This information can be notified to the user via the breast pump device.

When the breast pump is activated, optionally a measurement of the operating parameters of the pressure source, like a vacuum source can be performed. These measurements can be used as reference values.

According to an embodiment of the invention, the detector 140 may be coupled to a vacuum sensor to detect the vacuum created by the pressure source, like a vacuum source 120. According to an embodiment of the invention, after the detection of the presence of a breast in the breast receiving portion 130, the pressure source, like a vacuum source 120 may be operated at various operating modes by the controller 110. In particular, various suction profiles can be implemented by the pressure source, like a vacuum source 120. These suction profiles may be adapted over time.

During the detection mode, the controller 110 controls the pressure source, like a vacuum source 120 to generate a low vacuum. This is in particular advantageous as it allows the user to correctly place the breast inside the breast receiving portion 120. Once the breast detector 140 has detected the correct placement of the breast in the breast receiving portion, the controller 110 may initiate a stimulation mode of the pressure source, like a vacuum source 120.

According to an embodiment of the invention, the controller 110 may initiate the second operating mode (stimulation mode or expression mode of the vacuum source 120) even if the breast detector 140 has not detected the correct placement of a breast inside the breast receiving portion 130 after a predefined period of time. This is advantageous as it will allow the operation of the breast pump to extract milk even if the breast detector has not yet detected the correct placement of a breast inside the breast receiving portion. In other words, this feature allows a fallback operation of the breast pump.

According to an embodiment of the invention, the breast pump may comprise massaging expression kits which allow a massaging of the breast during the stimulation and extraction mode of the vacuum source.

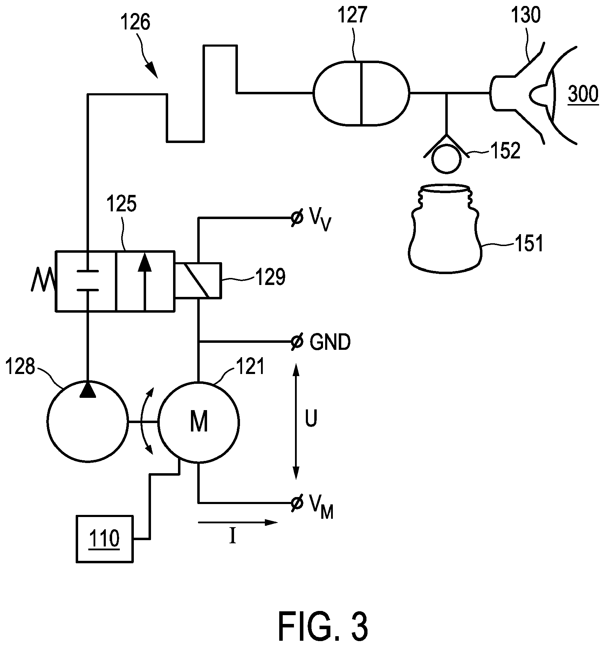

Fig. 3 shows a schematic representation of a breast pump device according to an embodiment of the invention. The breast pump device 100 according to the invention is an electric breast pump which comprises a funnel 130 as breast receiving portion, a motor 121 for driving a vacuum pump 128 as well as a release valve 129. The vacuum pump 128 is coupled to a membrane 127 via a tube 126. Moreover, a solenoid 129 is provided to drive the valve 125. A breast 300 of a user is placed into the funnel 130 (breast receiving portion) and the motor 121 is activated in order to create a vacuum in the funnel 130. The valve 152 is provided allowing air and milk to flow towards a milk storage container or milk bottle 151. The controller 110 controls the voltage of the motor 121 and the solenoid. The current I and/or the voltage V_{M} at the motor 121 can be detected.

The power of the motor 121 is calculated by a multiplication of the current and the voltage (P = U x I). If a load is present for the pump 128 then the amount of power which is required by the motor 121 will increase. If either the current or the voltage of the motor 121 is fixed, this will result in an increase of the other parameter. If either the current or the voltage is set at a low value, the increase of the other parameter will be more pronounced.

By detecting the increase of one of the parameters (current, voltage), the breast pump device can detect whether or not a breast has been placed into the funnel or the breast receiving portion. Accordingly, an automatic detection of the presence of a breast in the breast receiving portion is achieved. Hence, the breast detection is performed based on the monitoring of operation parameters of the pressure source, like a vacuum source 120.

According to an embodiment of the invention, the motor 121 can be operated in the region of a stall voltage of the motor. If for example the breast 300 is then placed into the funnel 130, then a load detection of the breast pump device can be performed. In order for the load detection, it is only required to monitor the current flowing through the motor 121. If a breast 300 is placed into the funnel 130, this will lead to a significant increase of the current of the motor 121. This significant current increase can be detected and the presence of a breast in the funnel or the breast receiving portion can be thus detected.

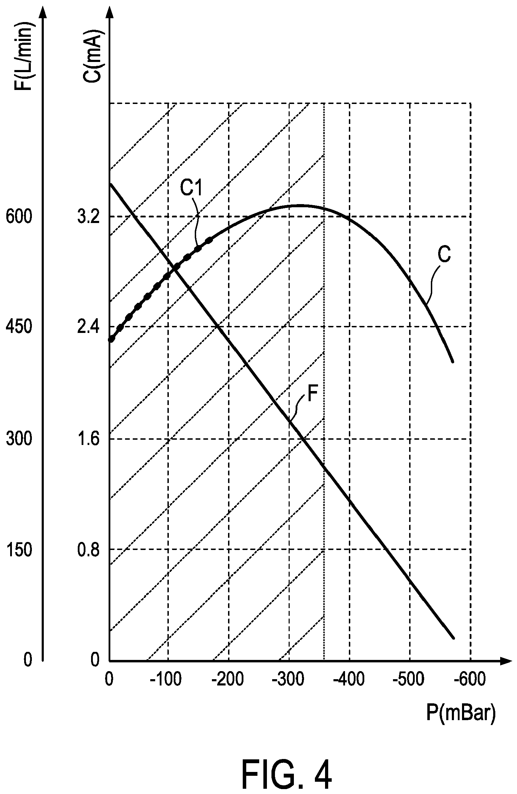

Fig. 4 shows a graph depicting a behavior of a vacuum pump according to an embodiment of the invention. In Fig. 4, the pressure P, the current C and the flow F are depicted. Fig. 4 in particular discloses the pump behavior in relation to the achieved pressure. As can be seen, with increasing pressure, the flow increases. Furthermore, with increasing pressure, the current will increase until a maximum and will then decrease again. According to an embodiment of the invention, only the first portion C1 of the current function C is used as this first portion C1 is substantially linear and therefore predictable. The breast detector 140 can be implemented by a current measurement or power measurement of the motor as described above.

According to a further embodiment of the invention, the breast detector may also use parameters other than the operating parameters of the pressure source, like a vacuum source to detect the presence of a breast. Hence, the motor rotation may be used by the breast detector. Alternatively, the breast detector may comprise a pressure sensor, a flow sensor, a capacitive sensor, a resistive sensor, an optical sensor, a mechanical sensor, a chemical sensor, a temperature sensor, an acoustic sensor, a magnetic sensor and/or a vital body signs sensor. Furthermore, the breast detector 140 may use a detection of human body in the breast receiving portion 130.

According to an embodiment of the invention, in a detection mode, the breast pump device may be operating in a stall running mode, i.e. the motor is operated in the region of the stall voltage such that the speed of the motor is reduced.

The breast pump according to the invention can be worked for open or closed systems (with or without balg or membrane 127). Accordingly, the breast pump can still be operated if the valve 125 and/or tube 126 are removed.

Other variations of the disclosed embodiment can be understood and effected by those skilled in the art in practicing the claimed invention from a study of the drawings, the disclosure and the appended claims.

In the claims, the word "comprising" does not exclude other elements or steps and in the indefinite article "a" or "an" does not exclude a plurality.

A single unit or device may fulfil the functions of several items recited in the claims. The mere fact that certain measures are recited in mutual different dependent claims does not indicate that a combination of these measurements cannot be used to advantage. A computer program may be stored/distributed on a suitable medium such as an optical storage medium or a solid state medium, supplied together with or as a part of other hardware, but may also be distributed in other forms such as via the internet or other wired or wireless telecommunication systems.

Any reference signs in the claims should not be construed as limiting the scope.

## Claims

1. Breast pump device (100), comprising
at least one breast receiving portion (130) configured to receive a breast of a user,
a pressure source (120) coupled to the at least one breast receiving portion (130) and being configured to generate an overpressure or an underpressure according to at least a first operating mode or a second operating mode,
a controller (110) configured to control an operation of the pressure source (120) according to the at least first operating mode or second operating mode, and
a breast detector (140) coupled to the controller (110) and being configured to detect a first state when no breast is placed in the at least one breast receiving portion (130) and to detect a second state when the breast is placed into the at least one breast receiving portion (130) and to communicate the first or second state to the controller (140),
wherein the controller (110) is configured to initiate an action, if the breast detector (140) has detected the second state.

2. Breast pump device (100), wherein the controller (110) is configured to change a parameter of the breast pump device or to output of at least one of a notification, a signal and a command, if the breast detector (140) has detected the second state.

3. Breast pump device according to claim 2, wherein the controller (110) is configured to operate the pressure source (120) in a first operating mode if the breast detector (140) has detected the first state or in a second operating mode if the breast detector (140) has detected the second state.

4. Breast pump device (100) according to claim 3, wherein the breast detector (140) is configured to detect the first or second state based on operating parameters of the pressure source (120).

5. Breast pump device (100) according to claim 4, wherein the operating parameters of the pressure source (120) are selected in such a way that a presence of a breast causes a detectable change in the operating parameter of the pressure source (120).

6. Breast pump device (100) according to claim 4, wherein the operating parameters of the pressure source (120) include speed or acceleration data of a motor (121) of the pressure source (120) or voltage or current measurements of the motor (121).

7. Breast pump device (100) according to claim 4, wherein the controller (110) is configured to operate the pressure source (120) in a detection mode as the first mode, wherein the pressure source (120) generates a low underpressure, and wherein the controller (110) is configured to operate the pressure source (120) in the second operating mode for milk expression

8. Breast pump device (100) according to claim 2, wherein the controller (110) is configured to initiate the second operating mode of the pressure source (120) based on an input from the user, based on an external signal or if the breast detector (140) has not detected the second state after a predefined period of time.

9. Breast pump device (100), comprising
at least one breast receiving portion (130) configured to receive a breast of a user,
a pressure source (120) coupled to the at least one breast receiving portion (130) and being configured to generate an overpressure or an underpressure according to at least a first operating mode or a second operating mode,
a controller (110) configured to control an operation of the pressure source (120) according to the first or second operating mode,
a breast detector (140) coupled to the controller (110) and being configured to detect a first state when no breast is placed in the at least one breast receiving portion (130) and to detect a second state when the breast is placed into the at least one breast receiving portion (130) or to detect a third state when the breast is not placed correctly into the breast receiving portion (130) such that leakage occurs based on operating parameters of the pressure source and to communicate the first, second or third state to the controller (140),
wherein the controller (110) is configured to operate the pressure source (120) in a first operating mode if the first state has been detected or a second operating mode if the second state has been detected,
wherein the controller (110) is configured to operate the pressure source (120) in a third operating mode if the third state has been detected.

10. A breast pump (100) according to claim 9, wherein the controller (110) is configured to change a parameter of the breast pump device and/or to output a notification if the breast detector has detected the third state.

11. Breast pump device (100) according to claim 9, wherein the breast detector is configured to detect the third state based on operating parameters of the pressure source (120).

12. Breast pump device (100) according to claim 1 or 9, wherein the controller (110) is configured to initiate a different sequence of modes or program than the original mode or sequence of modes after an interruption.

13. Method of operating a breast pump device (100) which has at least one breast receiving portion (130), a pressure source (120), a controller (110) and a breast detector (140), comprising the steps of
detecting whether a breast is placed into the breast receiving portion (130) by the breast detector (140) and communicating a first state to the controller when no breast is placed in the breast receiving portion or a second state when the breast has been detected inside the breast receiving portion,
changing a parameter of the breast pump device (100) and/or outputting a notification, signal or command, if the breast detector (140) has detected the second state.

14. Method of operating a breast pump device (100) according to claim 13, comprising the steps of:
detecting the first or second state based on the operating parameters of the pressure source (120),
operating the pressure source (120) in a first operating mode if the breast detector has detected the first state or in a second operating mode if the breast detector (140) has detected the second state.

15. A computer program for operating a breast pump, the computer program comprising program code means for causing a breast pump as defined in claim 1 to carry out the steps of the method of operating a breast pump as defined in claim 13, when the computer program is run on a computer controlling the breast pump.
